# EUROPÄISCHE PATENTSCHRIFT

(11) **EP 1 448 519 B1**
(45) Veröffentlichungstag und Bekanntmachung des Hinweises auf die Patenterteilung: **03.08.2011**
(21) Anmeldenummer: 02787689.5
(22) Anmeldetag: 15.11.2002
(51) Int. Cl.: C07D 207/26

(54) **VERFAHREN ZUR KONTINUIERLICHEN HERSTELLUNG VON N-METHYL-2-PYRROLIDON (NMP)**
METHOD FOR THE CONTINUOUS PRODUCTION OF N-METHYL-2-PYRROLIDONE (NMP)
PROCEDE DE PRODUCTION CONTINUE DE N-METHYLE-2-PYRROLIDONE (NMP)

(30) Priorität: 20.11.2001 DE 10156885
(43) Veröffentlichungstag der Anmeldung: 25.08.2004
(73) Patentinhaber: BASF SE, 67056 Ludwigshafen (DE)
(72) Erfinder: RUDLOFF, Martin, 67273 Weisenheim (DE); STOPS, Peter, 67122 Altrip (DE); HENKES, Erhard, 64683 Einhausen (DE); SCHMIDTKE, Helmut, 64625 Bensheim (DE); FISCHER, Rolf-Hartmuth, 69121 Heidelberg (DE); JULIUS, Manfred, 67117 Limburgerhof (DE); LEBKÜCHER, Rolf, 68165 Mannheim (DE); ROSS, Karl-Heinz, 67269 Grünstadt (DE)
(86) Internationale Anmeldenummer: PCT/EP2002/012804
(87) Internationale Veröffentlichungsnummer: WO 2003/053924

(56) Entgegenhaltungen:
- EP-A- 1 004 577
- WO-A-99/52867
- US-A- 3 775 431
- ELVERS B ET AL: "Ullmann's Encyclopedia of Industrial Chemistry, PASSAGE" POLY (VINYL ESTERS) TO REDUCTION, ULLMANN'S ENCYCLOPEDIA OF INDUSTRIAL CHEMISTRY, WEINHEIM, VCH, DE, Bd. A22, 1993, Seiten 458-459, XP002201293 in der Anmeldung erwähnt

## Beschreibung

Die vorliegende Erfindung betrifft ein Verfahren zur kontinuierlichen Herstellung von N-Methyl-2-pyrrolidon (NMP) durch Umsetzung von gamma-Butyrolacton mit Monomethylamin in der Flüssigphase.

NMP ist wegen bestimmter vorteilhafter Eigenschaften ein wichtiges, vielseitiges Lösungsmittel und Reaktionsmedium für die chemische Industrie (Ullmann's Encyclopedia of Industrial Chemistry, 5th Ed., Vol. A22, Seiten 458 bis 459).

Gemäß Ullmann's Encyclopedia of Industrial Chemistry, 5^{th} Ed., (siehe oben) wird NMP hauptsächlich durch Umsetzung von gamma-Butyrolacton (GBL) mit Methylamin in einem Schaftreaktor (Hochdruck-Rohr mit speziellen Leitblechen) bei 200 bis 350°C und ca. 10 MPa (= ca. 100 bar) hergestellt.

Winnacker, Küchler, "Chemische Technologie", 4. Aufl., 1982, Band 6, beschreibt auf Seite 99, Zeilen 5-9, dass gamma-Butyrolactam (2-Pyrrolidon) aus GBL durch vollständige Umsetzung mit flüssigem Ammoniak bei 270 °C und etwa 120 bar z.B. in einer Kaskade von drei Rührreaktoren hergestellt wird (Selektivität: 94 Mol%) (DE-A-17 95 007) und dass in analoger Umsetzung mit Methylamin NMP entsteht.

Chem. Abstracts 77:61805 und Derwent Abstr. 40726T-E
(JP-B4-47 021 420) betrifft die Herstellung von NMP durch Umsetzung von GBL mit MeNH₂ in Gegenwart von Wasser. Die diskontinuierliche Umsetzung von GBL mit MeNH₂ und Wasser im Molverhältnis 1 : 2 : 2-4 bei 250°C (2-1,5 h) ergibt NMP in 99 % Ausbeute. Ohne Gegenwart von Wasser bei der Synthese liegt die Ausbeute nur bei 96 % (1,5 h).

Chem. Abstracts 124:145893 (CN-A-110 46 35) beschreibt die Synthese von NMP durch Umsetzung von GBL mit MeNH₂ bei 220 - 290°C und ≥ 6 MPa (≥ 60 bar). Die diskontinuierliche Umsetzung einer Mischung von GBL, 30 %igem wässrigen MeNH₂ und Wasser im Gewichtsverhältnis 1 : 1,4 : 5,6 bei 280°C und 6 MPa (60 bar) ergibt NMP in 97 % Ausbeute. Das obige Gewichtsverhältnis entspricht einem Molverhältnis GBL : MeNH₂ : H₂O von 1 : 1,2 : 26,7 (ohne Berücksichtigung des Wassers der MeNH₂-Lösung) bzw. einem Molverhältnis von 1 : 1,2 : 31,5 (mit Berücksichtigung des Wassers der MeNH₂-Lösung).

Chem. Abstracts 129:67694 (JP-A2-10 158 238) betrifft die Umsetzung von GBL mit 1,03 bis 1,50 Moläquivalenten MeNH₂ in Gegenwart 1,0 bis 2,9 Moläquivalenten Wasser bei 250 bis 300 °C. Die diskontinuierliche Umsetzung von GBL mit MeNH₂ und Wasser im Molverhältnis 1 : 1,1 : 1 bei 280°C (1 h) ergibt NMP in 99,9 % Ausbeute.

Derwent Abstract 97-234193/21 (RO-B1-111 189) beschreibt einen zweistufigen kontinuierlichen Prozess zur Herstellung von NMP aus GBL und MeNH₂ in Gegenwart von Wasser.
Die Reaktionsbedingungen in der ersten Stufe sind: Molverhältnis von GBL, MeNH₂ und Wasser = 1 : 1,2 : 2,1, (0,5 bis 2) x 10⁴ N/m² (= 0,05 bis 0,2 bar (Überdruck)) und 40 bis 50 °C.
Die Reaktionsbedingungen in der zweiten Stufe sind: 280 bis 290°C und (90 bis 100) x 10⁴ N/m² (= 9 bis 10 bar (überdruck)).

Derwent Abstract 1998-607722, Chem. Abstracts 134:178463 (RO-B1-113 640) betrifft ein Verfahren zur kontinuierlichen Herstellung von NMP in flüssiger Phase in zweistufiger Fahrweise.
Die Reaktionsbedingungen in der ersten Stufe sind: Molverhältnis von GBL, MeNH₂ und Wasser = 1 : 1,2 : 2,1, 150 bis 170°C und (90 bis 100) x 10⁵ N/m² (= 90 bis 100 bar).
Die Reaktionsbedingungen in der zweiten Stufe sind: 280 bis 290°C und (90 bis 100) x 10⁵ N/m² (= 90 bis 100 bar).

WO 99/52867 (Pantochim S.A.) offenbart ein Verfahren zur kontinuierlichen Herstellung von NMP durch Umsetzung von GBL mit Monomethylamin in der Flüssigphase in drei aufeinanderfolgenden Reaktionsstufen, wobei die erste Stufe bei Temperaturen zwischen 150 und 220°C, die zweite Stufe bei Temperaturen zwischen 220 und 270°C und die dritte Stufe bei Temperaturen zwischen 250 und 310°C betrieben wird. Der Druck in allen drei Stufen liegt zwischen 30 und 90 ATE (30,4 und 91,2 bar), bevorzugt zwischen 40 und 60 ATE (40,5 und 60,8 bar). Das Molverhältnis von GBL zu Monomethylamin beträgt 1 : 1,05 bis 1 : 1,4.

Chem. Abstract 82:139947 (JP-B4-49 020 585) beschreibt die Umsetzung von einem Teil GBL mit zwei Teilen Monomethylamin und 2 bis 4 Teilen Wasser (= Molverhältnis von 1 : 5,5 : 9,6-19,1) bei 250°C (2 h) zu NMP in 99 % Ausbeute. Der Temperaturbereich für die Umsetzung beträgt im allgemeinen 200 bis 300 °C, insbesondere 230 bis 300°C.

Chem. Abstract 87:5802 (JP-A2-49 041 364) offenbart die Umsetzung von GBL mit Monomethylamin und Wasser als 1 : 1,4 : 4 Mischung (= Molverhältnis von 1 : 3,9 : 19,1) bei 250 °C und 45 bis 50 kg/cm² (= 44 bis 49 bar).

US-A-2,964,535 (Monsanto) betrifft ein Verfahren zur Reinigung von NMP durch Behandlung mit einem Alkalimetallhydroxid in wässriger Lösung und anschließender Destillation.

Erfindungsgemäß wurde erkannt, dass sich aus dem Stand der Technik unter anderem folgende Nachteile ergeben:
a) niedrige Raum-Zeit-Ausbeuten an NMP,
b) der Einsatz von größeren Mengen Wasser in der Synthese, der u.a. bei der destillativen Aufarbeitung des Reaktionsaustrags zu hohen Energiekosten führt,
c) der große molare Überschuss an Monomethylamin bezogen auf GBL, der einen hohen technischen Aufwand bei der Aufarbeitung des Reaktionsaustrags und Rückgewinnung des unumgesetzten Monomethylamins und damit hohe Investitions- und Energiekosten bedingt, und
d) der hohe technische Aufwand verbunden mit hohen Investitions- und Betriebskosten bei einer Durchführung der Umsetzung in mehreren Reaktionsstufen, z. B. gemäß WO 99/52867.

Der vorliegenden Erfindung lag die Aufgabe zugrunde, unter Überwindung der Nachteile des Stands der Technik, ein verbessertes, selektives Verfahren zur Herstellung von NMP in hohen Ausbeuten und Raum-Zeit-Ausbeuten und hoher Qualität (z. B. Reinheit > 99,5 Gew.-%, APHA-Farbzahl ≤ 50, GBL-Restgehalt < 0,05 Gew.-%) aufzufinden.

Demgemäß wurde ein Verfahren zur kontinuierlichen Herstellung von N-Methyl-2-pyrrolidon (NMP) durch Umsetzung von gamma-Butyrolacton (GBL) mit Monomethylamin (MMA) in der Flüssigphase gefunden, welches dadurch gekennzeichnet ist, dass man GBL und MMA im Molverhältnis von 1 : 1,08 bis 1 : 1,3 einsetzt und die Umsetzung bei einer Temperatur von 340 bis 380°C und einem Absolutdruck von 70 bis 120 bar durchführt, wobei das Einsatzstoffgemisch weniger als 10 gew% wasser enthält.

Vorzugsweise wird die erfindungsgemäße Umsetzung bei einer Temperatur von 350 bis 370°C, z. B. 360°C, durchgeführt.

Die erfindungsgemäße Umsetzung wird vorzugsweise bei einem Absolutdruck von 70 bis 110 bar, insbesondere bei einem Absolutdruck von 80 bis 110 bar, ganz besonders bei einem Absolutdruck von 80 bis 105 bar, z. B. 90 bar, durchgeführt.

Das Molverhältnis der Einsatzstoffe GBL : Monomethylamin beträgt im erfindungsgemäßen Verfahren 1 : (1,08 bis 1,3), ganz besonders 1 : (1,08 bis 1,2), ganz besonders bevorzugt 1 : (1,08 bis 1,15), z. B. 1 : 1,12.

Das Einsatzstoffgemisch enthält weniger als 10 Ges.-%, ganz besonders weniger als 5 Gew.-%, insbesondere weniger als 1 Gew.-%, Wasser. In einer besonders bevorzugten Ausführungsform enthält das Einsatzstoffgemisch kein Wasser.

Das erfindungsgemäße Verfahren kann z. B. wie folgt ausgeführt werden:
Als Reaktor dient vorzugsweise ein aufrecht stehendes, schlankes Hochdruckrohr, das nach einem Wärmetauscher mit Umgang mit einem über einen Druckregler gesteuerten Entspannungsventil ausgestattet ist. Die Feedströme werden vorzugsweise vom Reaktoraustrag vorgeheizt, so dass im Reaktor durch die Exothermie die erfindungsgemäßen Reaktionstemperaturen eingestellt werden können. Im Reaktor sind bevorzugt mehrere Siebböden zur Verhinderung einer Rückvermischung und besseren Ausbildung eines Pfropfenströmung (quasi Rührkesselkaskade) eingebaut. Die Anzahl der Siebböden kann z. B. 10 bis 40, bevorzugt 20 bis 30, betragen.

Flüssiges Monomethylamin, gegebenenfalls gemischt mit zurückgewonnenem Monomethylamin aus der Aufarbeitung (siehe unten), wird mit einer Pumpe, z. B. Membranpumpe, über einen Wärmetauscher W2 und über einen dampfbetriebenen Aufheizer W3 an das untere Ende des Rohrreaktors gepumpt (bevorzugte Sumpffahrweise).

GBL wird mit einer Pumpe, z. B. Membranpumpe, über einen dampfbetriebenen Aufheizer W1 ebenfalls an das untere Ende des Rohrreaktors geführt, wobei eine Durchmischung der Einsatzstoffe stattfindet und die Einsatzstoffe GBL und Monomethylamin im oben angegebenen, erfindungsgemäßen Molverhältnis vorliegen.

Das flüssige Monomethylamin und das GBL werden in einer besonders bevorzugten Ausführungsform dem Rohrreaktor im Zentrum des Reaktorbodens durch einen Zweistoff-Injektor separat zugeführt. Während das GBL hierbei mit hoher Strömungsgeschwindigkeit (bevorzugt 4 - 12 m/s) durch die Zentraldüse eingeleitet wird, gelangt das flüssige Monomethylamin an der Außenseite dieser Düse durch einen Ringspalt in den Reaktor. Die Edukte werden hierbei als Treibstrahl in ein Umlaufrohr geleitet, das sich im Eintrittsbereich des Reaktors befindet, ca. ein Drittel des freien Rohrquerschnittes umschließt und eine Länge von bevorzugt 1,5 bis 2,0 m besitzt. Die Rezirkulation von Flüssigkeit, die an der Außenseite des Umlaufrohres erfolgt und durch den Treibstrahl in Gang gehalten wird, führt zu einem intensiven ersten Kontakt der Reaktionspartner im oben angegebenen, erfindungsgemäßen Molverhältnis.

Im Reaktor erfolgt unter den oben angegebenen Temperaturen und Drucken die kontinuierliche Umsetzung des GBLs mit Monomethylamin in flüssiger Phase in exothermer Reaktion zu NMP.

Die Umsetzung wird bevorzugt in Abwesenheit eines Katalysators durchgeführt.

Die mittleren Verweilzeiten des Reaktionsgemischs im Reaktor bzw. in allen Reaktoren, wenn mehrere eingesetzt werden (vergl. weiter unten), ermittelt mit der Dichte von GBL und Monomethylamin (flüssig) bei Normaltemperatur (20°C), betragen je nach Belastung im allgemeinen 10 bis 60 Min., bevorzugt 15 bis 30 Min.

Die Raum-Zeit-Ausbeuten an NMP im Reaktorrohaustrag betragen im allgemeinen ≥ 1 kg NMP / (h ● l_{Reaktor}), besonders 1 bis 3 kg NMP / (h ● l_{Reaktor}), ganz besonders 1 bis 2,5 kg NMP / (h ● l_{Reaktor}) z. B. 2 kg NMP / (h ● l_{Reaktor}).

(l_{Reaktor} = Reaktorvolumen in Liter, bei mehreren Reaktoren Gesamtreaktorvolumen in Liter).

Das erhaltene Reaktionsprodukt wird aus dem Reaktor kontinuierlich als Zulauf in eine Destillationskolonne K1 entspannt, wobei der Reaktoraustrag zunächst zur Aufheizung des Methylamins ganz oder teilweise über den Wärmetauscher W2 (siehe oben) geleitet und dabei abgekühlt wird.

Dem Zulauf der Kolonne K1 kann zuvor Kopfdestillat der Destillationskolonne K2 (Hauptbestandteil: NMP. Siehe unten) zugemischt werden.

In der Destillationskolonne K1 werden, z. B. bei 40 bis 240°C und 1 bis 2 bar, noch vorhandenes Monomethylamin und Wasser abdestilliert. Die Energiezufuhr kann mit einem Umlaufverdampfer erfolgen. Das Destillat ist eine im allgemeinen 15 bis 30 Gew.-%ige, besonders 15 bis 25 Gew.-%ige, z. B. ca. 20 Gew.-%ige, wässrige Monomethylaminlösung. Aus der Lösung kann mit bekannten Verfahren wasserfreies Monomethylamin zurückgewonnen werden.

Bevorzugt erfolgt die Abdestillation des noch vorhandenen Monomethylamins und des Wassers in dieser Destillationsstufe in Gegenwart von 0,05 bis 1 Gew.-%, besonders 0,05 bis 0,2 Gew.-%, ganz besonders 0,08 bis 0,15 Gew.-%, (jeweils bezogen auf die Menge an NMP im Zulauf dieser Destillationsstufe) Hydroxiden der Metalle Na, K, Li, Ba oder Ca.

Besonders bevorzugt erfolgt die Abdestillation des MMA/Wasser-Gemischs in dieser Destillationsstufe in Gegenwart von NaOH, das als Natronlauge mittels einer Pumpe in den Zulauf der Kolonne K1 dosiert wird. Z. B. wird hier wässrige 25 %ige Natronlauge so eingesetzt, dass die Konzentration an NaOH 0,05 bis 0,2 Gew.-%, insbesondere 0,08 bis 0,15 Gew.-%, (jeweils bezogen auf die Menge an NMP im Zulauf dieser Destillationsstufe) beträgt.

Erfindungsgemäß wurde erkannt, dass durch diese besondere Verfahrensausgestaltung im Reaktionsprodukt enthaltene saure Komponenten (welche Korrosionsprobleme in den Apparaten bereiten können) und ggf. restliche Spuren an unumgesetzten GBL gebunden werden (Umsetzung des GBLs zum entsprechenden Metallsalz der gamma-Hydroxybuttersäure), so dass schließlich ein besonders reines NMP erhalten wird.

Anschließend wird der Sumpf aus der Kolonne K1 entnommen, gegebenenfalls abgekühlt und mit einer Pumpe zur Kolonne K2 zwecks Reindestillation des NMPs befördert. In der Kolonne K2 wird reines NMP als flüssiger Seitenabzug gewonnen. Die Sumpftemperaturen betragen in der Regel 100 bis 140°C, bei 0,01 bis 0,02 bar. (Siedepunkt von NMP: 204°C / 1 bar). Die Wärmezufuhr kann auch hier mit einem Umlaufverdampfer erfolgen. Das Kopfdestillat, das neben NMP MMA und Wasser enthält, kann zur Kolonne K1 (siehe oben) und/ oder zum Reaktoreingang zurückgeführt werden.

Nach dem erfindungsgemäßen Verfahren wird NMP nach Destillation in Ausbeuten > 97 %, insbesondere > 97,5 %, ganz besonders > 98 % erhalten.

Der Umsatz an GBL beträgt im allgemeinen > 99 %, insbesondere > 99,5 %, ganz besonders > 99,9 %.

Die Selektivität (bezogen auf GBL) beträgt im allgemeinen > 97 %, insbesondere > 98 %, ganz besonders > 99 %.

Das erfindungsgemäß erhaltene NMP besitzt nach Destillation eine hohe Qualität:
Die Reinheit beträgt im allgemeinen > 99 Gew.-%, insbesondere ≥ 99,5 Gew.-%, ganz besonders ≥ 99,8 Gew.-%.

Der Gehalt an GBL beträgt im allgemeinen < 0,05 Gew.-%, insbesondere < 0,02 Gew.-%, der Gehalt an Monomethylamin < 50 ppm, insbesondere < 20 ppm (ppm-Angaben bezogen auf Gewichtsteile).

Die APHA-Farbzahl nach DIN ISO 6271 des erfindungsgemäß erhaltenen NMPs beträgt im allgemeinen ≤ 50, insbesondere ≤ 20.

Das erfindungsgemäße Verfahren kann auch in einer Apparatur (Rohrreaktor), wie sie in der Schrift DE-A-17 95 007, auf die hier diesbezüglich ausdrücklich Bezug genommen wird, beschrieben ist, ausgeführt werden.

Das erfindungsgemäße Verfahren wird einstufig, d.h. in einem Reaktor, der auch aus apparatetechnischen Gründen in zwei oder mehrere Apparate (Reaktoren) aufgeteilt sein kann, wobei dann in jedem dieser Reaktoren die erfindungsgemäßen Bedingungen bezüglich Druck und Temperatur herrschen, durchgeführt.

### Beispiele

Die NMP-Synthese wurde unter den unten genannten Bedingungen in kontinuierlicher Fahrweise in einem Rohrreaktor (RA4, Länge x Innendurchmesser = 2000 mm x 30 mm, Ringspalt 9 mm, Volumen = 1,1 1, elektrisch beheizt) durchgeführt. Der Reaktor wurde dabei in Sumpffahrweise im geraden Durchgang betrieben. Die Einsatzstoffe Monomethylamin (MMA) und GBL wurden vorgeheizt und mittels Pumpen zum Reaktoreingang gefördert, wo die Mischung der beiden Ströme erfolgte. Der Gesamtwassergehalt der Einsatzstoffe betrug weniger als 1 Gew.-%.

Am Reaktorausgang befand sich ein Hochdruckabscheider aus welchem die Gasphase (Stickstoff und Inerte) als Abgas entspannt wurde. Die sich abscheidende Flüssigphase wurde in ein Gefäß auf Atmosphärendruck entspannt und anschließend überschüssiges MMA und Reaktionswasser als Gemisch abdestilliert.

| | |
|---|---|
| Temperatur: | 360°C |
| Druck: | 90 bar (Druck durch N₂ gehalten) |
| Belastung: | 1,4 kg GBL / (l_{Reaktor} ● h) |
| GBL : MMA - Molverhältnis | 1 : 1,1 |
| Abgas | 20 N1 / h |

| | |
|---|---|
| [Nl = Normliter = auf Normalbedingungen umgerechnetes Volumen; Reaktorbelastung in kg GBL pro Liter Reaktorvolumen und Stunde]. | |

In der folgenden Tabelle ist die Zusammensetzung der Reaktionsausträge (nach Monomethylamin-Abdampfung) aufgeführt.

**Tabelle: GC-Analyse der Rohausträge aus der NMP-Synthese**

| Versuch | NMP | GBL | DMP *) | Sonstige |
|---|---|---|---|---|
| Nr. | [GC-Fl.%] | [GC-Fl.%] | [GC-Fl.%] | [GC-Fl.%] |
| 1 | 99,5 | 0,02 | 0,15 | 0,33 |
| 2 | 99,6 | 0,01 | 0,19 | 0,2 |

| | | | | |
|---|---|---|---|---|
| GC-Bedingungen: 30 m DB-1, Temperaturprogramm: 80°C Eingangstemperatur, 4°C/Min. Aufheizrate, 250°C Endtemperatur. Der H₂O-Restgehalt ist nicht berücksichtigt. *) DMP = 1,3-Dimethyl-2-pyrrolidon und 1,4-Dimethyl-2-pyrrolidon (entstanden aus alpha- und beta-Methyl-GBL, das jeweils als Verunreinigung im eingesetzten GBL vorhanden war). Die NMP-Ausbeute im Rohaustrag in Bezug auf das eingesetzte GBL betrug > 99 %. Nach Reindestillation wurde das NMP mit einer Reinheit > 99,8 Gew.-% gewonnen. Die Raum-Zeit-Ausbeute an NMP im Reaktorrohaustrag betrug 1,5 kg NMP / (h ● l_{Reaktor}). | | | | |

## Patentansprüche

1. Verfahren zur kontinuierlichen Herstellung von N-Methyl-2-pyrrolidon (NMP) durch Umsetzung von gamma-Butyrolacton (GBL) mit Monomethylamin (MMA) in der Flüssigphase, **dadurch gekennzeichnet, dass** man GBL und MMA im Molverhältnis von 1 : 1,08 bis 1 : 1,3 einsetzt und die Umsetzung einstufig bei einer Temperatur von 340 bis 380°C und einem Absolutdruck von 70 bis 120 bar durchführt, wobei das Einsatzstoffgemisch weniger als 10 Gew.-% Wasser enthält.

2. Verfahren nach Anspruch 1, **dadurch gekennzeichnet, dass** man die Umsetzung bei einer Temperatur von 350 bis 380°C durchführt.

3. Verfahren nach einem der vorhergehenden Ansprüche, **dadurch gekennzeichnet, dass** man die Umsetzung bei einem Absolutdruck von 80 bis 110 bar durchführt.

4. Verfahren nach einem der vorhergehenden Ansprüche, **dadurch gekennzeichnet, dass** man GBL und MMA im Molverhältnis von 1 : 1,08 bis 1 : 1,2 einsetzt.

5. Verfahren nach einem der vorhergehenden Ansprüche, **dadurch gekennzeichnet, dass** man die Umsetzung in einem aufrecht stehenden Rohrreaktor durchführt.

6. Verfahren nach dem vorhergehenden Anspruch, **dadurch gekennzeichnet, dass** man die Umsetzung in Sumpffahrweise durchführt.

7. Verfahren nach einem der beiden vorgehenden Ansprüche, **dadurch gekennzeichnet, dass** man das Monomethylamin und das GBL dem Rohrreaktor im Reaktorboden durch einen Zweistoff-Injektor separat zuführt.

8. Verfahren nach einem der vorhergehenden Ansprüche, **dadurch gekennzeichnet, dass** man die Umsetzung in Abwesenheit eines Katalysators durchführt.

9. Verfahren nach einem der vorhergehenden Ansprüche, **dadurch gekennzeichnet, dass** man nach der Umsetzung aus dem Reaktionsprodukt zunächst Wasser und Monomethylamin und schließlich das N-Methyl-2-pyrrolidon abdestilliert.

10. Verfahren nach einem der vorhergehenden Ansprüche, **dadurch gekennzeichnet, dass** man nach der Umsetzung aus dem Reaktionsprodukt das Wasser und Monomethylamin in Gegenwart von Hydroxiden der Metalle Na, K, Li, Ba oder Ca, insbesondere in Gegenwart von NaOH, abdestilliert.

11. Verfahren nach einem der vorhergehenden Ansprüche, **dadurch gekennzeichnet, dass** man das N-Methyl-2-pyrrolidon in einer Destillationskolonne als flüssigen Seitenabzug gewinnt.

12. Verfahren nach einem der vorhergehenden Ansprüche zur Herstellung von N-Methyl-2-pyrrolidon mit einer Selektivität von > 98 %.

13. Verfahren nach einem der vorhergehenden Ansprüche zur Herstellung von N-Methyl-2-pyrrolidon mit einer Reinheit von ≥ 99,5 %.

14. Verfahren nach einem der vorhergehenden Ansprüche zur Herstellung von N-Methyl-2-pyrrolidon mit einer APHA-Farbzahl ≤ 20.

15. Verfahren nach einem der vorhergehenden Ansprüche zur Herstellung von N-Methyl-2-pyrrolidon mit einer Raum-Zeit-Ausbeute von ≥ 1 kg NMP / (h ● l_{Reaktor}).

## Claims

1. A process for the continuous preparation of N-methyl-2-pyrrolidone (NMP) by reacting gammabutyrolactone (GBL) with monomethylamine (MMA) in the liquid phase, wherein GBL and MMA are used in a molar ratio of from 1:1.08 to 1:1.3 and the reaction is carried out in a single stage at from 340 to 380°C and an absolute pressure of from 70 to 120 bar, where the feed mixture comprises less than 10% by weight of water.

2. The process according to claim 1, wherein the reaction is carried out at from 350 to 380°C.

3. The process according to either of the preceding claims, wherein the reaction is carried out at an absolute pressure of from 80 to 110 bar.

4. The process according to any of the preceding claims, wherein GBL and MMA are used in a molar ratio of from 1:1.08 to 1:1.2.

5. The process according to any of the preceding claims, wherein the reaction is carried out in an upright tube reactor.

6. The process according to the preceding claim, wherein the reaction is carried out in the upflow mode.

7. The process according to either of the two preceding claims, wherein the monomethylamine and the GBL are fed separately into the tube reactor at the bottom of the reactor via a two-fluid injector.

8. The process according to any of the preceding claims, wherein the reaction is carried out in the absence of a catalyst.

9. The process according to any of the preceding claims, wherein firstly water and monomethylamine and finally the
N-methyl-2-pyrrolidone are distilled off from the reaction product after the reaction.

10. The process according to any of the preceding claims, wherein the water and monomethylamine are distilled off from the reaction product in the presence of hydroxides of the metals Na, K, Li, Ba or Ca, in particular in the presence of NaOH, after the reaction.

11. The process according to any of the preceding claims, wherein the N-methyl-2-pyrrolidone is isolated as a liquid stream from a side offtake of a distillation column.

12. The process according to any of the preceding claims for preparing N-methyl-2-pyrrolidone with a selectivity of > 98%.

13. The process according to any of the preceding claims for preparing N-methyl-2-pyrrolidone having a purity of a 99.5%.

14. The process according to any of the preceding claims for preparing N-methyl-2-pyrrolidone having an APHA color number of ≤ 20.

15. The process according to any of the preceding claims for preparing N-methyl-2-pyrrolidone in a space-time yield of si kg NMP/(h ● l_{reactor}).

## Revendications

1. Procédé de fabrication continue de N-méthyl-2-pyrrolidone (NMP) par mise en réaction de gammabutyrolactone (GBL) avec de la monométhylamine (MMA) en phase liquide, **caractérisé en ce que** la GBL et la MMA sont utilisées en un rapport molaire de 1:1,08 à 1:1,3 et la réaction est réalisée en une étape à une température de 340 à 380 °C et à une pression absolue de 70 à 120 bar, le mélange de matières premières contenant moins de 10 % en poids d'eau.

2. Procédé selon la revendication 1, **caractérisé en ce que** la réaction est réalisée à une température de 350 à 380 °C.

3. Procédé selon l'une quelconque des revendications précédentes, **caractérisé en ce que** la réaction est réalisée à une pression absolue de 80 à 110 bar.

4. Procédé selon l'une quelconque des revendications précédentes, **caractérisé en ce que** la GBL et la MMA sont utilisées en un rapport molaire de 1:1,08 à 1:1,2.

5. Procédé selon l'une quelconque des revendications précédentes, **caractérisé en ce que** la réaction est réalisée dans un réacteur tubulaire vertical.

6. Procédé selon la revendication précédente, **caractérisé en ce que** la réaction est réalisée en mode de fond.

7. Procédé selon l'une quelconque des deux revendications précédentes, **caractérisé en ce que** la monométhylamine et la GBL sont introduites séparément dans le réacteur tubulaire dans le fond du réacteur par un injecteur à deux composants.

8. Procédé selon l'une quelconque des revendications précédentes, **caractérisé en ce que** la réaction est réalisée en l'absence d'un catalyseur.

9. Procédé selon l'une quelconque des revendications précédentes, **caractérisé en ce qu'**après la réaction, l'eau et la monométhylamine sont tout d'abord distillées du produit réactionnel, et enfin la N-méthyl-2-pyrrolidone.

10. Procédé selon l'une quelconque des revendications précédentes, **caractérisé en ce qu'**après la réaction, l'eau et la monométhylamine sont distillées du produit réactionnel en présence d'hydroxydes des métaux Na, K, Li, Ba ou Ca, notamment en présence de NaOH.

11. Procédé selon l'une quelconque des revendications précédentes, **caractérisé en ce que** la N-méthyl-2-pyrrolidone est obtenue dans une colonne de distillation en tant que sortie latérale liquide.

12. Procédé selon l'une quelconque des revendications précédentes, pour la fabrication de N-méthyl-2-pyrrolidone avec une sélectivité > 98 %.

13. Procédé selon l'une quelconque des revendications précédentes, pour la fabrication de N-méthyl-2-pyrrolidone avec une pureté ≥ 99,5 %.

14. Procédé selon l'une quelconque des revendications précédentes, pour la fabrication de N-méthyl-2-pyrrolidone avec un indice de coloration Alpha ≤ 20.

15. Procédé selon l'une quelconque des revendications précédentes, pour la fabrication de N-méthyl-2-pyrrolidone avec un rendement espace-temps ≥ 1 kg NMP / (h·l_{réacteur}).
